**Europäisches Patentamt**

**European Patent Office**

**Office européen des brevets**

(19)

(11) Publication number : **0 616 810 A1**

(12)

# EUROPEAN PATENT APPLICATION

(21) Application number : **94301204.7**

(22) Date of filing : **21.02.94**

(51) Int. Cl.⁵ : **A61K 35/78**

(30) Priority : **19.02.93 JP 53294/93**

(43) Date of publication of application :
**28.09.94 Bulletin 94/39**

(84) Designated Contracting States :
**DE ES FR GB IT**

(71) Applicant : **SOKEN Co., Ltd.**
**2216-Utazu Cho**
**Ayauta Gun, Kagawa Ken (JP)**

(72) Inventor : **Tokuyama, Takashi**
**2212 Utazu-cho**
**Ayauta-gun, Kagawa-ken (JP)**

(74) Representative : **Bubb, Antony John Allen et al**
**GEE & CO.**
**Chancery House**
**Chancery Lane**
**London WC2A 1QU (GB)**

(54) **Anti-cancer agent.**

(57)    A composition which is effective in preventing or treating cancer comprises ground germinated rice or a liquid extract of rice or germinated rice. The extract is obtained by treatment with water or an organic solvent, optionally in conjunction with an enzyme treatment or fermentation.
    The anti-ulcer agent may be administered orally.

EP 0 616 810 A1

The present invention relates to an agent for the prophylaxis and treatment of cancer, the agent being obtained from rice or germinated rice and capable of being used in the fields of medicine and food.

The most usual cause of death in Japan at present is cancer, and various medicaments have been developed for its prophylaxis and treatment.

However, these medicaments pose problems in respect of their side effects, and restrictions on dosage and the dosage period. Also, since these medicaments are almost always mixtures of single compounds problems arise from the side effects of each single compound and furthermore the taking of a medicament for a long period of time causes anxiety. An agent, which is effective for the prophylaxis and treatment of cancer, exhibits no side effects, and is safe has not yet been developed.

On the other hand, uses of rice have been developed such as for producing 'sake', 'shochu' (Japanese spirits distilled from rice), sweet sake, vinegar and 'koji' (malted rice), in addition to its use as a staple food, and thus rice has long been an indispensable part of life. As another known use of rice, there may be cited the use of a small rice-bran bag for cosmetic purposes. Accordingly rice has been considered to be at most a simple staple food providing a starch source, and there has been neither the conception that rice contains a therapeutically effective component nor that this effective component might be utilized.

Currently, the side effects of medicaments on human beings have become a problem and hence there has been desire for a cancer prophylactic and treatment agent which has no side effects and which is safe, even when the agent is regularly used.

An object of the present invention is to provide a cancer prophylactic and treatment agent obtained from rice or germinated rice, which is safe and inexpensive and which is also utterly safe when the agent is regularly used.

The inventors have investigated various vegetable components centring around rice which is a staple food from the view point of an environmental balance between animals and plants; and during the course thereof, it has become apparent that rice has many possibilities and effects which have never been anticipated until now. Thus, rice which has been used as a stapie food and the great safety of which has thus been actually proved has been targeted as the subject of the present investigation into its general utilization. During the course of the investigations, it has been discovered that rice and germinated rice contain a component exhibiting activity for the prophylaxis and treatment of cancer.

The nature of the component exhibiting such prophylactic and treatment activity has not yet been clarified, but it has been confirmed that the forms of rice and germinated rice described below do show a prophylactic and treatment effect on cancer:-

(1) A ground product of germinated rice; or a material containing the ground product.

(2) An extract of rice or germinated rice; or a material containing the extract.

(3) A product obtained by effecting an enzymatic decomposition or 'koji' treatment of the hydrated product of rice or germinated rice; or a material containing the foregoing product.

(4) An extract of rice or germinated rice which has been subjected to enzymatic decomposition or 'koji' treatment before extraction, during extraction or after extraction; or a material containing the extract.

(5) A product obtained by alcoholic-fermentation of organic acid-fermentation of an extract of rice or germinated rice or by alcohol-fermentation or organic acid-fermentation of the product obtained by subjecting rice or germinated rice to enzymatic decomposition or 'koji' treatment; or a material containing the fermentation product.

In the present invention the term "rice" means any rice harvested not only in Japan but also in other countries, including unpolished rice such as non-glutinous rice or glutinous rice, and polished rice regardless of the breed and the kind thereof. Furthermore, not more than 92% of a polished rice bran formed by polishing rice can be used as "rice" in this invention, and such a polished rice can be used as "rice" in this invention, and such a polished rice bran is inexpensive and economical. Also, germinated rice may be used in the present invention.

In addition, since the effective component of rice or germinated rice is stable to heat and light, the raw materials described above may be subjected to a surface denaturation such as dipping, cooking, broiling (including sand broiling, netting broiling and hot blast broiling), cook broiling and lyophilization; light denaturation such as an UV irradiation; press broiling such as pat rice; or a raw material treatment such as frying. Also, the effect of the components is not changed by applying thereto the foregoing treatments.

Rice and germinated rice can be used effectively as they are; however, from the view point of practical use, it is preferred to use them as the ground products. For powdering rice and germinated rice by grinding, a general method using a grinder or a rice-cleaning machine may be used.

To germinate rice, rice with embryo buds is immersed in water or water is sprayed onto rice with embryo buds in order to cause it to germinate. The germination temperature is from 5°C to 70°C. However, if rice germinates, there are no restrictions on the temperature and the time of germination. Also, if there is a possibility of the water putrefying during the germination, it is preferred to renew the water to prevent it from putrefying or to apply an antiseptic agent.

In this invention, the term "germination" means any stage of the germination process of rice from directly before germination to fully germinated rice. Germinated rice is used after being washed well and in this case, the washed germinated rice may be dried.

If, in the case of the extraction of rice or germinated rice, or during the enzymatic decomposition or 'koji' treatment of rice, or germinated rice, the raw material rice, or germinated rice, is ground into a granular form or a powder form, the surface area of the raw material is increased to improve efficiency. The raw material need not be ground, but in this case a long time is required for the decomposition or the extraction of the rice tissue.

In the case of extracting rice or germinated rice with water, efficiency is improved by increasing the extraction temperature, but even at a low temperature the extraction can be carried out sufficiently. However, when the extraction temperature is lower than 40°C, it is preferable that the pH of the system be adjusted to an acidic or an alkaline range, or than an antiseptic or an alcohol be added to the system to prevent the rice or germinated rice from rotting.

The extraction time may be long or short providing that the effective component is extracted, and may be determined according to the extraction temperature. Also, the extraction may be carried out under increased pressure, at normal pressure or under reduced pressure.

In the case of the water extraction of rice or germinated rice, the most troublesome problem is the phenomenon of gelatinization. If rice is gelatinized, not only is the extraction efficiency reduced but also the practical extraction operation becomes very difficult. To prevent the occurrence of gelatinization of the rice, the extraction may be carried out with the addition of amylase or the system may be acidified with hydrochloric acid to cut the starch. By employing the foregoing method the problem can be solved adequately, and there is no practical problem is encountered in employing the method.

Since the effective component in the extracted product may be stable in the presence of an acid or an alkali, acidic decomposition extraction or alkaline decomposition extraction can be carried out. In this case, if necessary, neutralization and desalting can be performed subsequently.

Even in the case of extraction with an organic solvent, it is desirable to grind or powder the rice or powder the rice or germinated rice as fine as possible during extraction. As the organic solvent, a general organic solvent such as, for example, ethanol, acetone, n-hexane or methanol, can be used but since any organic solvent which is noxious to humans must be completely removed from the extract, the use of an organic solvent that is not noxious to humans is preferable.

Also, the rice or germinated rice may be subject to an enzymatic decomposition or 'koji' treatment. The term "enzyme decomposition" in this invention means that one or more kinds of enzymes are caused to act on the rice or germinated rice, such as an amylolytic enzyme, a proteolytic enzyme, lipase, a fibre decomposition enzyme, a lignin decomposition enzyme or a pectin decomposition enzyme.

Also, there are no restrictions on the type of 'koji' moulds or the strain and kind of rice used for producing 'koji'. Furthermore, when carrying out the extraction described above, the foregoing enzyme decomposition or 'koji' treatment may be performed before, during or after the extraction.

In the present invention, when an alcohol fermentation or an organic acid fermentation such as, for example, a lactic acid fermentation or an acetic acid fermentation, is applied simultaneously with or after the treatment described above, the treatment becomes more effective as described below.

First, when alcohol fermentation is applied, concentration of the treated product is facilitated, enabling the effective component to be easily concentrated. Also, lactic acid fermentation improves the flavour of the product in the case of using the product in, for example, drinks and by applying the acetic acid fermentation, the product can be utilized as a seasoning liquid such as vinegar. Thus, by utilizing organic acid fermentation, the product finds wide application.

The product of the present invention obtained as described above may be used as it is without separation of residues or may be used after compression and/or filtration. When the product is used as it is, it should be subjected to sterilization or removal of fungi. In addition, the product may be dried and used, for example, as granules or tablets. Furthermore, the product can be used after compounding with various foods.

The test methods for determining the anti-cancer activity of the products of the present invention and the results thereof are described hereinbelow.

**Test Method 1**

After being fasted, from the morning male F344 rats (average weight 180 g, aged 8 weeks) were divided into groups of five, one in each gauge; and in the evening 1 ml of water was administered to each rat of the control group, and 1 ml of the product of the present invention was administered to each rat of the test groups through a stomach tube. After 30 minutes, as a secondary administration, 1 ml of water was administered to each rat of the control group and 1 ml of an aqueous 2.3 M sodium chloride solution was administered to each rat of the positive control group and to each rat of the test groups. One pellet diet was fed to each rat as a bait. After 17 hours, the stomach mucous membrane (pyloric gland mucous membrane) was extracted from each rat, an organ culture was carried out, and the composite DNA synthesis was measured. That is [³H]thymidine was added to the culture liquid, after cultivating for 2 hours, the DNA synthesis was extracted from the tissue, and [³H]thymidine taken in DNA was determined by a liquid scintillation counter.

The results obtained are shown in Table 1.

## Table 1

| Product in Example | (A) | (B) | (C) | (D) | (E) |
|---|---|---|---|---|---|
| Control | Water | 1 ml | Water | 1 ml | 430 ± 175 |
| Positive Control | Water | 1 ml | NaCl soln.* | 1 ml | 3960 ± 1683 |
| Example 1 | Product | 1 g | " | 1 ml | 980 ± 515 |
| Example 2 | Product | 1 ml | " | 1 ml | 910 ± 385 |
| Example 4 | Product | 1 ml | " | 1 ml | 610 ± 302 |
| Example 4** | Product | 1 ml | " | 1 ml | 700 ± 318 |
| Example 5 | Product | 1 ml | " | 1 ml | 586 ± 180 |
| Example 6 | Product | 1 ml | " | 1 ml | 783 ± 381 |
| Example 8 | Product | 1 ml | " | 1 ml | 760 ± 319 |
| Example 10 | Product | 1 ml | " | 1 ml | 975 ± 210 |
| Example 12 | Product | 1 ml | " | 1 ml | 1080 ± 532 |
| Example 14 | Product | 1 ml | " | 1 ml | 993 ± 480 |
| Example 16 | Product | 1 ml | " | 1 ml | 1110 ± 562 |
| Example 18 | Product | 1 ml | " | 1 ml | 928 ± 410 |
| Example 20 | Product | 1 ml | " | 1 ml | 1152 ± 562 |
| Example 22 | Product | 1 ml | " | 1 ml | 896 ± 425 |
| Example 24 | Product | 1 ml | " | 1 ml | 416 ± 103 |
| Example 26 | Product | 1 ml | " | 1 ml | 486 ± 150 |
| Example 28 | Product | 1 ml | " | 1 ml | 498 ± 114 |
| Example 30 | Product | 1 ml | " | 1 ml | 338 ± 128 |
| Example 32 | Product | 1 ml | " | 1 ml | 300 ± 130 |
| Example 34 | Product | 1 ml | " | 1 ml | 398 ± 200 |
| Example 4[*3] | Ext.1 | 1 ml | " | 1 ml | 3990 ± 200 |
| Example 4[*4] | Ext.2 | 1 ml | " | 1 ml | 1105 ± 215 |

In Table 1:

      (A):      Primary administered material.

      (B):      Dose of the primary administered material.

      (C):      Secondary administered material.

      (D):      Dose of the secondary administered material.

      (E):      $[^3H]$Thymidine($/\mu g$-DNA).

      (*):      Aqueous sodium chloride solution.

      (**):      Example 4 was followed using glutinous rice.

      (*3):      Unpolished rice bran extract obtained as in Example 4.

      (*4):      Unpolished rice extract obtained as in Example 4.

      (Ext.1):  Unpolished rice bran extract.

      (Ext.2):  Unpolished rice extract.

As shown in Table 1, it can be seen that the amount of $[^3H]$thymidine per µg of the replication DNA synthesis is $430 \pm 175$ for the control but is $3960 \pm 1680$ for the positive control, which is about 10 times that of the control.

On the other hand, in the groups administered with the products of the present invention, the synthesis of replication DNA was restrained in all the cases. It was also confirmed that the foregoing effect was more improved by combining the extraction of rice or germinated rice and the enzymatic decomposition thereof or the product obtained by the 'koji' treatment thereof; and further, in the case of applying the alcohol fermentation or the organic acid fermentation, an excellent effect was obtained. Also, as shown in the case of using the product obtained in Example 4, the product obtained from polished rice exhibited a greater effect than the product obtained from unpolished rice, and in the case of using the product obtained from unpolished rice bran, the effect was not observed.

Accordingly, the reason that the product from unpolished rice exhibits a lesser effect than the product from polished rice is that unpolished rice contains rice bran and it is considered that the existence of this rice bran exerts a bad influence.

Common salt (sodium chloride) is considered to be a promotor for a stomach carcinogenicity and the products of the present invention clearly restrain the action thereof, which shows that the products of the present invention are effective as anti-cancer agents.

**Test Method 2**

After orally administering a carcinogenic substance, N-methyl-N'-nitro-N-nitrosoguanidine (MNNG) to male F344 rats aged 4 weeks, divided into groups of five, at a dose of 150 mg/kg-weight a day per one rat for 8 weeks to make the rats cancerate, each product of the present invention was orally administered to the rats for 50 weeks in place of drinking water.

After 50 weeks, the stomach pyloric gland mucous membrane was extracted from each rat, an organ culture was carried out, and the composite DNA synthesis was measured. The measurement method was same as in Test Method 1, $[^3H]$-thymidine was determined, and the results are shown in Table 2 below.

In addition, ordinary food and water, as drinking water, were administered to a control group. Also, MNNG and water, as drinking water, were administered to a positive control group.

To confirm the carcinogenic property of MNNG, $[^3H]$thymidine was measured after 8 weeks on the rats of each of the control and the positive control groups.

The results are shown in Table 2.

## Table 2

| | $[^3H]$Thymidine ( /$\mu$g-DNA) |
|---|---|
| Control | 493 ± 182 |
| Positive Control | 5104 ± 1528 |
| Product in Example 1 | 2921 ± 913 |
| Product in Example 2 | 2513 ± 1021 |
| Product in Example 4 | 1622 ± 872 |
| Product* in Example 4 | 1492 ± 911 |
| Product in Example 5 | 892 ± 516 |
| " 6 | 2001 ± 808 |
| " 8 | 1826 ± 936 |
| " 10 | 923 ± 612 |
| " 12 | 1823 ± 722 |
| " 14 | 1529 ± 621 |
| " 16 | 1743 ± 701 |
| " 18 | 1721 ± 690 |
| " 20 | 1611 ± 633 |
| " 22 | 1232 ± 521 |
| " 24 | 721 ± 367 |
| " 26 | 743 ± 403 |
| " 28 | 792 ± 312 |
| " 30 | 539 ± 202 |
| " 32 | 572 ± 243 |
| " 34 | 621 ± 243 |
| Product** in Example 4 | 2846 ± 899 |
| Control after 8 weeks | 452 ± 143 |
| Positive Control after 8 weeks | 4921 ± 162.5 |

Note: The product in Example 1 was administered as a mixture with food.

In Table 2:

Product is the product of the present invention.

(*): Example 4 was followed using glutinous rice.

(**): Example 4 was followed using unpolished rice.

It is well known that MNNG is a substance injurious to normal genes in the stomach of a rat, causing cancer. By feeding MNNG to a rat at 150 mg/kg-weight per day for 8 weeks, the rat is cancerated and the abnormal growth of genes occurs. If the abnormal growth of genes is continued, the cancer gene also grows and the number of the cancer genes becomes about $10^6$, ie they become a cancer cell. As seen in Table 2, [³H]thymidine is $452 \pm 143$ ($\mu$g-DNA) for the control after 8 weeks, while it is $4921 \pm 1625$ for the positive control (after 8 weeks) administered with MNNG, and hence it is clear that by the administration of MNNG, the composite DNA synthesis is considerably increased.

However, it has been confirmed that by administering the product of the present invention, the increased composite DNA synthesis is clearly decreased in all the products of this invention, which shows that the products of the present invention have an excellent anti-cancer activity for restraining the abnormally grown genes.

The present invention is next described further in the following Examples.

Example 1

By immersing 1 kg of rice with germs in water of 25°C for 3 days, rice was germinated. The germinated rice, after washing well, was dried at 50°C for 24 hours and finely ground to provide 990 g of the product of the present invention

Example 2

Polished rice was ground in a grinder to provide 500 g of the ground product. To the ground product was added 1,500 ml of water and, after lowering pH thereof with hydrochloric acid, the mixture was allowed to stand for 10 days. Thereafter, the mixture was squeezed by a squeezer and the clear liquid obtained was neutralized to provide 1,200 ml of the product of the present invention and 760 g of residues.

Example 3

A further 1,190 ml of product of the present invention was obtained by following the same procedure as in Example 2 using 500 g of the product obtained in Example 1.

Example 4

Polished rice was ground in a grinder to provide 500 g of the ground product. To the ground product were added 10 g of a liquefied enzyme and 1,500 ml of water. Thereafter, the temperature of the mixture was gradually raised and after extracting under boiling for 5 minutes, the extract was cooled. Then, the extract was squeezed by a squeezer to provide 1,420 ml of the product of the present invention and 560 g of residues.

Example 5

A further 1,400 ml of product of the present invention was obtained by following the same procedure as in Example 4 using 500 g of the product obtained in Example 1.

Example 6

Polished rice was ground in a grinder to provide 500 g of the ground product. To the ground product was added 1,500 ml of an aqueous 2N sodium hydroxide solution and the resultant mixture was allowed to stand for 5 days. Thereafter, the mixture was squeezed by a squeezer to provide 1,350 ml of a clear liquid and 650 g of residues. By neutralizing the clear liquid with 10N hydrochloric acid, 1,480 ml of the product of the present invention was obtained.

Example 7

A further 1,490 ml of product of the present invention was obtained by following the same procedure as in Example 6 using 500 g of the product obtained in Example 1.

Example 8

Polished rice was ground in a grinder to provide 500 g of the ground product. To the ground product was added 1,500 ml of 95% ethanol and the mixture was allowed to stand for 5 days. Thereafter, the mixture was squeezed by a squeezer to provide 1,300 ml of the clear liquid and 650 g of residues. To the clear liquid was added 2,000 ml of water and the mixed liquid was concentrated by a rotary evaporator to provide 1,500 ml of the product of the present invention.

Example 9

A further 1,500 ml of product of the present invention was obtained by following the same procedure as in Example 8 using 500 g of the product obtained in Example 1.

Example 10

Polished rice was ground in a grinder to provide 500 g of ground product. To the ground product were added 300 g of 'koji' and 1,500 ml of water and the mixture was allowed to stand for 20 hours at 55°C. Thereafter, the mixture was squeezed in a squeezer to provide 1,230 ml of the product of the present invention and 1,000 g of residues.

Example 11

A further 1,230 ml of product of the present invention was obtained by following the same procedure as Example 10 using 500 g of the product obtained in Example 1.

Example 12

Polished rice was ground by a grinder to provide 500 g of ground product. To the ground product were added 2 g of proteolytic enzyme and 1,500 ml of water and the resultant mixture was allowed to stand at 50°C for 2 hours. Thereafter, the mixture was squeezed by a squeezer to provide 1,310 ml of the product of the present invention and 670 g of residues.

Example 13

A further 1,380 ml of product of the present invention was obtained by following the same procedure as in Example 12 using 500 g of the product obtained in Example 1.

Example 14

Polished rice was ground in a grinder to provide 500 g of the ground product. To the ground product were added 2 g of lipase and 1,500 ml of water and the resultant mixture was allowed to stand at 50°C for 20 hours. Thereafter, the mixture was squeezed by a squeezer to provide 1,290 ml of the product of the present invention and 680 g of residues.

Example 15

A further 1,360 ml of the product of the present invention was obtained by following the same procedure as in Example 14 using 500 g of the product obtained in Example 1.

Example 16

Polished rice was ground in a grinder to provide 500 g of the ground product of polished rice. To the ground product were added 2 g of a fibre decomposition enzyme and 1,500 ml of water and the resultant mixture was allowed to stand at 50°C for 20 hours. Thereafter, the mixture was squeezed by a squeezer to provide 1,330 ml of the product of the present invention and 650 g of residues.

### Example 17

A further 1,370 g of product of the present invention was obtained by following the same procedure as in Example 16 using 500 g of the product obtained in Example 1.

### Example 18

Polished rice was ground in a grinder to provide 500 g of the ground product. To the ground product were added 2 g of an amylolytic enzyme and 1,500 ml of water and the resultant mixture was allowed to stand at 55°C for 20 hours. Thereafter, the mixture was squeezed by a squeezer to provide 1,380 ml of the product of the present invention and 600 g of residues.

### Example 19

A further 1,400 ml of product of the present invention was obtained by following the same procedure as in Example 18 using 500 g of the product obtained in Example 1.

### Example 20

Polished rice was ground in a grinder to provide 500 g of the ground product. To the ground product were added 2 g of a pectin decomposition enzyme and 1,500 ml of water and the resultant mixture was allowed to stand at 50°C for 20 hours. Thereafter, the mixture was squeezed by a squeezer to provide 1,320 ml of the product of the present invention and 660 g of residues.

### Example 21

A further 1,300 ml of product of the present invention was obtained by following the same procedure as in Example 20 using 500 g of the product obtained in Example 1.

### Example 22

Polished rice was ground in a grinder to provide 500 g of the ground product of polished rice. To the ground product were added 2 g of a proteolytic enzyme, 2 g of lipase, 2 g of a fibre decomposition enzyme, 2 g of an amylolytic enzyme, 2 g of a pectin decomposition enzyme, and 1,500 ml of water and the resultant mixture was allowed to stand at 50°C for 20 hours. Thereafter, the mixture was squeezed by a squeezer to provide 1,420 ml of the product of the present invention and 560 g of residues.

### Example 23

A further 1,440 ml of product of the present invention was obtained by following the same procedure as in Example 22 using 500 g of the product obtained in Example 1.

### Example 24

By following the same procedure as in Example 22, 2,000 g of the enzyme decomposition product of polished rice was obtained. Thereafter, the temperature thereof was gradually raised and after extracting under boiling for 5 minutes, the extract was cooled. Thereafter, the extract was squeezed by a squeezer to provide 1,400 ml of the product of the present invention and 550 g of residues.

### Example 25

A further 1,420 ml of product of the present invention was obtained by following the same procedure as in Example 24 using 500 g of the product obtained in Example 1.

Example 26

Polished rice was ground in a grinder to provide 500 g of the ground product. To the ground product were added 300 g of 'koji' and 1,500 ml of 40% ethanol and the resultant mixture was allowed to stand at 55°C for 48 hours. Thereafter, the mixture was squeezed by a squeezer to provide 1,300 ml of a clear liquid and 850 g of residues. Then, to the clear liquid was added 1,000 ml of water and the mixture was concentrated by a rotary evaporator to provide 1,300 ml of the product of the present invention.

Example 27

A further 1,300 ml of product of the present invention was obtained by following the same procedure as in Example 26 using 500 g of the product obtained in Example 1.

Example 28

By following the same procedure as in Example 4, 2,000 g of the extract of polished rice was obtained. To the extract were added 2 g of a proteolytic enzyme, 2 g of lipase, 2 g of a fibre decomposition enzyme, 2 g of an amylolytic enzyme, and 2 of a pectin decomposition enzyme and the resultant mixture was allowed to stand at 50°C for 24 hours. Thereafter, the mixture was squeezed by a squeezer to provide 1,400 ml of the product and 580 g of residues.

Example 29

A further 1,390 ml of product of the present invention was obtained by following the same procedure as in Example 28 using 500 g of the product obtained in Example 1.

Example 30

By following the same procedure as in Example 24, 2,000 g of the enzyme decomposition extract of polished rice was obtained. To the enzyme decomposition extract was added yeast and an alcohol fermentation was carried out. Thereafter, the fermentation product was squeezed in a squeezer to provide 1,880 ml of the product of the present invention and 80 g of residues.

Example 31

A further 1,800 ml of product of the present invention was obtained by following the same procedure as in Example 30 using 500 g of the product obtained in Example 1.

Example 32

By following the same procedure as in Example 24, 2,000 g of the enzyme decomposition extract of polished rice was obtained. After sterilizing the enzyme decomposition extract by boiling, the extract was cooled to 37°C, and 200 ml of a starter previously prepared by culturing a lactic acid bacteria was added thereto followed by stirring well. Thereafter, the mixture was sealed tightly and the lactic acid fermentation was carried out at 37°C for 2 days. Then, the fermentation product was squeezed in a squeezer to provide 1,380 ml of the product and 590 g of residues.

Example 33

A further 1,400 ml of product of the present invention was obtained by following the same procedure as in Example 32 using 500 g of the product obtained in Example 1.

Example 34

To 1,000 ml of the product of this invention obtained in Example 24 was added 80 ml of 95% ethanol and the acetic acid fermentation was carried out for 20 hours. Thereafter, the fermentation product was filtrated to provide 990 ml of the product of the present invention.

11

Example 35

A further 1,000 ml of product of the present invention was obtained by following the same procedure as in Example 34 using 500 g of the product obtained in Example 1.

Next, Examples of the preparation of tablets by compounding the product of the present invention, and the preparation of a soft drink using the product of the present invention, are described below. The present invention is not limited to the compounding Examples.

Example 36 (Tablet)

By drying 100 g of the product obtained in Example 24 by freeze-drying, 20 g of the dried product was obtained. By using 10 g of the dried product, tablets were prepared as described below.

| | |
|---|---|
| Product of the invention | 10 g |
| Polyethylene glycol 6000 | 10 g |
| Sodium laurylsulfate | 1.5 g |
| Corn starch | 3 g |
| Milk sugar | 25 g |
| Magnesium stearate | 0.5 g |

After weighing each component described above, polyethylene glycol 6000 was heated to a temperature of from 70°C to 80°C and after mixing it with the product of this invention, sodium laurylsulfate, corn starch, and milk sugar, the mixture was cooled as it was. The solidified mixture was granulated in a grinder. Then, after mixing the granules with magnesium stearate, the mixture was tableted to form tablets each having a weight of 250 mg.

Example 37 (Soft Drink)

In this Example, the product of this invention obtained in Example 22 was used.

| | |
|---|---|
| Product of the invention | 15% by weight |
| Licorice extract | 0.01% " |
| Sugar | 4% " |
| Lemon juice | 2.5% " |
| Purified water | 78.49% " |

By mixing the above components in an ordinary manner, a soft drink was obtained.

As described in detail above, there is obtained according to the present invention an excellent agent for the prophylaxis and treatment of cancer, which is easily administered, is utterly safe, and has its effect through continuous eating and/or drinking.

Since rice has until now been a staple food, production processes for products of rice other than food and uses of rice in fields other than food have scarcely developed. Furthermore, as rice has long been used as a staple food its safety has been sufficiently proved. With the present invention, not only has a very excellent cancer prophylactic and treatment agent been discovered, but it is also very significant that a new use of rice has been found, and the consumption of rice can be increased by the promotion of rice where it is currently said to be in surplus production.

**Claims**

1. A composition comprising germinated rice, or an extract of rice or germinated rice, for use in medicine.

2. A composition comprising germinated rice, or an extract of rice or germinated rice, for use in the prophylaxis or treatment of cancer.

3. A composition as claimed in Claim 1 or 2, wherein said germinated rice is in ground or particulate form, or said extract is derived from rice or germinated rice in ground or particulate form.

4. A composition as claimed in Claim 1, 2 or 3, wherein the extract is obtained by hydrating polished rice or germinated rice, allowing to stand at a temperature between room temperature and 40°C, and separating the liquid product.

5. A composition as claimed in Claim 1 or 2, wherein the extract is obtained by hydrating polished rice or germinated rice, pre-treating a mixture of the hydrated product and water with amylase or 'koji', maintaining the mixture at a temperature of from 40°C to boiling, and separating the liquid product.

6. A composition as claimed in Claim 1 or 2, wherein the extract is obtained by treating polished or germinated rice with either an acidic aqueous solution or an alkaline aqueous solution, allowing the mixture to stand for several days to form a liquid product, and separating the liquid product.

7. A composition as claimed in Claim 1 or 2, wherein the extract is obtained by hydrating polished rice or germinated rice, subjecting the hydrated product to treatment with at least one enzyme selected from amylolytic enzymes, proteolytic enzymes, lipases, fibre decomposition enzymes, lignin decomposition enzymes, pectin decomposition enzymes and 'koji', and either separating the liquid product so obtained or boiling the mixture and then separating the liquid product.

8. A composition as claimed in Claim 1 or 2, wherein the extract is obtained by hydrating polished rice or germinated rice, allowing the hydrated product to stand at a temperature of from room temperature to 40°C, subjecting the product to treatment with at least one enzyme selected from amylolytic enzymes, proteolytic enzymes, lipases, fibre decomposition enzymes, lignin decomposition enzymes, pectin decomposition enzymes and 'koji', and separating the liquid product.

9. A composition as claimed in Claim 1 or 2, wherein the extract is obtained by treating polished rice or germinated rice with an organic solvent, and separating the liquid product.

10. A composition as claimed in Claim 1 or 2, wherein the extract is obtained by treating polished rice or germinated rice with an organic solvent, subjecting the mixture so formed to treatment with at least one enzyme selected from amylolytic enzymes, proteolytic enzymes, lipases, fibre decomposition enzymes, lignin decomposition enzymes, pectin decomposition enzymes and 'koji', and separating the liquid product.

11. A composition as claimed in any one of Claims 3 to 10, wherein the liquid product is subjected to an alcohol fermentation or an organic acid fermentation.

12. The use, in the manufacture of a medicament for the prophylaxis or treatment of cancer, of a composition as claimed in any preceding claim.

**European Patent Office**

## EUROPEAN SEARCH REPORT

Application Number

EP 94 30 1204

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| Y | EP-A-0 027 514 (DAICEL CHEMICAL INDUSTRIES, LTD) * page 2, line 6 - page 3, line 2 * | 1-12 | A61K35/78 |
| Y | DATABASE WPI Section Ch, Week 9237, Derwent Publications Ltd., London, GB; Class B04, AN 92-304664 & JP-A-4 210 645 (SO-KEN KK) 31 July 1992 * abstract * | 1-12 | |
| A | PATENT ABSTRACTS OF JAPAN vol. 015, no. 409 (C-0876)18 October 1991 & JP-A-03 168 054 (MITSUO KODA) 19 July 1991 * abstract * | | |
| A | DATABASE WPI Section Ch, Week 9213, Derwent Publications Ltd., London, GB; Class B04, AN 92-102783 & JP-A-4 049 240 (CHIBA SEIFUN KK) 18 February 1992 * abstract * | | TECHNICAL FIELDS SEARCHED (Int.Cl.5) A61K |

The present search report has been drawn up for all claims

| Place of search | Date of completion of the search | Examiner |
|---|---|---|
| THE HAGUE | 30 May 1994 | Rempp, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)